(19) Europäisches Patentamt
European Patent Office
Office européen des brevets

(11) EP 4 206 134 A1

(12) EUROPEAN PATENT APPLICATION
published in accordance with Art. 153(4) EPC

(43) Date of publication:
**05.07.2023 Bulletin 2023/27**

(21) Application number: **21860470.0**

(22) Date of filing: **26.08.2021**

(51) International Patent Classification (IPC):
***C01B 39/08*** *(2006.01)* ***C07C 69/68*** *(2006.01)*
***B01J 29/89*** *(2006.01)*

(52) Cooperative Patent Classification (CPC):
**B01J 29/89; B01J 35/10; B01J 37/10; B82Y 40/00; C01B 37/00; C01B 39/08; C07C 67/00; C07C 69/68;** Y02P 20/52

(86) International application number:
**PCT/CN2021/114731**

(87) International publication number:
**WO 2022/042636 (03.03.2022 Gazette 2022/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.08.2020 CN 202010888283**

(71) Applicants:
- **China Petroleum & Chemical Corporation**
  **Beijing 100728 (CN)**
- **Research Institute Of Petroleum Processing, Sinopec**
  **Beijing 100083 (CN)**

(72) Inventors:
- **XIA, Changjiu**
  **Beijing 100083 (CN)**
- **LIU, Yujia**
  **Beijing 100083 (CN)**
- **PENG, Xinxin**
  **Beijing 100083 (CN)**
- **LIN, Min**
  **Beijing 100083 (CN)**
- **ZHU, Bin**
  **Beijing 100083 (CN)**
- **SHU, Xingtian**
  **Beijing 100083 (CN)**

(74) Representative: **Hoffmann Eitle**
**Patent- und Rechtsanwälte PartmbB**
**Arabellastraße 30**
**81925 München (DE)**

# (54) TIN-TITANIUM-SILICON MOLECULAR SIEVE, PREPARATION METHOD THEREFOR AND USE THEREOF

(57) The present invention relates to a tin-titanium-silicon molecular sieve, a preparation method and an application thereof. The electron binding energy of framework tin active centers in the tin-titanium-silicon molecular sieve is 488.5 eV or less. In the tin-titanium-silicon molecular sieve, the molar ratio of titanium to silicon is preferably 0.005-0.03, and the molar ratio of tin to silicon is preferably 0.005-0.025. The tin-titanium-silicon molecular sieve of the invention has more catalytic active centers, a lower electron binding energy of framework tin active centers, and an excellent catalytic performance.

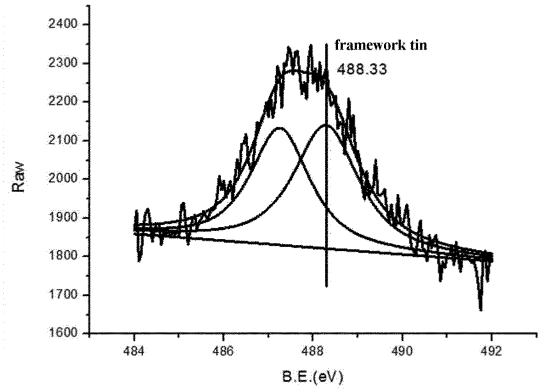


Figure 1

EP 4 206 134 A1

## Description

### TECHNICAL FIELD

The present invention relates to a tin-titanium-silicon molecular sieve, a preparation method and an application thereof.

### BACKGROUND TECHNOLOGY

In recent years, during the industrial production, heterogeneous catalysts have attracted a great attention due to their advantages such as reusability and easy separation. In the environmentally friendly conversion of oxygen-containing hydrocarbons, heteroatomic molecular sieves show incomparable advantages over conventional methods. Taramasso et al. synthesized TS-1 molecular sieve for the first time in 1983. People successively insert other heteroatoms (such as Fe, Sn, V, Zr and Ga etc.) into the molecular sieve topology, and prepare a variety of heteroatomic molecular sieves. In Sn-containing molecular sieves, tetra-coordinated tin atoms are uniformly inserted into the molecular sieve framework space structure through the isomorphous substitution, endows the molecular sieves with the unique acid catalytic performance, and the molecular sieves become a heteroatomic molecular sieve catalytic material with an important potential industrial application value. The empty orbit of the framework tetra-coordinated tin atom can accept lone pair electrons, and has strong activation properties for oxygen-containing functional groups. Therefore, tin-silicon molecular sieves exhibit a good catalytic performance in the solid Lewis acid catalytic reaction. They are often used in the saccharide isomerization reaction, the reaction for preparation of lactic acid and the derivatives thereof, the Baeyer-Villiger reaction and the Meerwein-Ponndorf-Verley Oxidation reaction.

Common tin-silicon molecular sieves include Sn-MFI, Sn-BEA, Sn-MEL, Sn-MWW, Sn-USY etc. In 1994, Ramaswamy et al. first synthesized an Sn-MFI molecular sieve by a hydrothermal method. The Sn-MFI molecular sieve not only has the acid catalytic function of tin, but also has the shape selection function of the ZSM-5 molecular sieve. However, the inorganic tin sources used in the synthesis of the tin-silicon molecular sieve by the hydrothermal method are all acidic and has a certain impact on the alkaline environment of the molecular sieve synthesis. As a result, molecular sieves cannot crystallize when a lot of tin sources are fed. Therefore, the tin-silicon molecular sieve synthesized by the hydrothermal method contains a limited number of tetra-coordinated framework tin active centers.

To overcome this problem, researchers have developed a post-insertion method to synthesize tin-silicon molecular sieves in recent years. Li et al. (The Journal of Physical Chemistry C, 2011, 115(9):3663-3670) fully contacted a dealuminated Beta molecular sieve with $SnCl_4$ vapor at 673-773K, which can synthesize an Sn-Beta molecular sieve with a tin content up to 6.1 wt%. Hammond et al. (Angewandte Chemie International Edition, 2012, 51(47):11736-11739) mixed tin acetate and dealuminated Beta molecular sieve evenly, and then calcined at a high temperature to obtain an Sn-Beta molecular sieve with a high tin content. Although the post-insertion method can prepare tin-silicon molecular sieves with a high framework tin content, there are still many problems during synthesis using the post-insertion method: the type and the number of framework defects are randomly generated in the dealumination process, so it is quite difficult to eliminate all hydroxyl defects by supplementing tin atoms, resulting in a poor synthesis repeatability; the post-inserted tin atoms are randomly distributed on the molecular sieve framework, rather than completely located in the thermodynamic stable position, so the framework tin is apt to lose during the reaction process, resulting in a poor activity stability; tin atoms may be deposited on the surface of molecular sieve particles, resulting in its inability to be evenly inserted into the framework of the tin-silicon molecular sieve and the production of $SnO_2$ aggregates.

### CONTENTS OF THE INVENTION

It is an object of the present invention to provide a tin-titanium-silicon molecular sieve, a preparation method and an application thereof. The tin-titanium-silicon molecular sieve of the invention has more catalytic active centers and an excellent catalytic performance.

To achieve the above object, a first aspect of the invention provides a tin-titanium-silicon molecular sieve. The electron binding energy of the framework tin active center in the tin-titanium-silicon molecular sieve is 488.5 eV or less.

Optionally, in the tin-titanium-silicon molecular sieve, the molar ratio of titanium to silicon is 0.005-0.03, and/or the molar ratio of tin to silicon is 0.005-0.025.

Optionally, the electron binding energy of the framework tin active center is 488.0-488.4 eV.

Optionally, the electron binding energy of the framework titanium active center in the tin-titanium-silicon molecular sieve is 460.0-461.2 eV.

Optionally, the chemical shift data of phosphorus corresponding to double-quantum nuclear magnetic framework tin species adsorbed by a trimethylphosphine probe molecule in the tin-titanium-silicon molecular sieve are <_ -23 ppm.

Optionally, the chemical shift data of phosphorus corresponding to double-quantum nuclear magnetic framework

titanium species adsorbed by a trimethylphosphine probe molecule in the tin-titanium-silicon molecular sieve are >_ -34 ppm.

Optionally, the external specific surface area of the tin-titanium-silicon molecular sieve is 100-150 $m^2/g$, and the total pore volume is 0.25-0.35 $cm^3/g$.

Optionally, the tin-titanium-silicon molecular sieve is an Sn-Ti-MFI molecular sieve or an Sn-Ti-MEL molecular sieve.

Optionally, the Sn-Ti-MEL molecular sieve has a structure in which long-strip grains overlap and stack, the grains preferably having a length of 10-50 nm, and a width of 10-30 nm.

A second aspect of the invention provides a method for preparing the tin-titanium-silicon molecular sieve provided by the first aspect of the invention, said method comprising:

S1, mixing an organic silicon source, an organic base, a solvent, a tin source, a titanium source and an alkaline template to obtain a first mixture, wherein the organic base is tetrapropyl ammonium hydroxide and/or tetrabutyl ammonium hydroxide; the molar ratio of the amounts of the organic silicon source, the organic base, the solvent, the tin source and the titanium source is 1: (0.05-0.6): (10-30): (0.005-0.04): (0.005-0.04), wherein the organic silicon source is calculated based on $SiO_2$, the tin source is calculated based on $SnO_2$, the titanium source is calculated based on $TiO_2$; the molar ratio of the amounts of the tin source and the alkaline template is 1: (1-10), wherein the tin source is calculated based on anion;
S2, heating the first mixture at 30-80 °C for 2-10 hours to obtain a second mixture;
S3, subjecting the second mixture to a hydrothermal reaction at 120-200 °C for 2-7 days to obtain a third mixture;
S4, taking out a solid in the third mixture and subjecting it to drying and calcination.

Optionally, the molar ratio of the amounts of the tin source and the alkaline template is 1: (4-8).

Optionally, the molar ratio of the amounts of the organic silicon source, the organic base, the solvent, the tin source and the titanium source is 1: (0.1-0.4): (15-30): (0.01-0.03): (0.01-0.03).

Optionally, step S1 comprises: mixing the organic silicon source, the organic base, the solvent and the titanium source to obtain a fourth mixture, mixing the resulting fourth mixture with the tin source and stirring for 0.5-2 hours, and then adding the alkaline template to obtain the first mixture.

Optionally, the organic silicon source has a structure shown in formula (I) below,

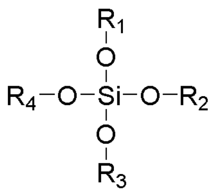

Formula (I)

In formula (I), $R_1$, $R_2$, $R_3$ and $R_4$ are each independently selected from C1-C4 alkyl, such as C1-C4 linear alkyl and C3-C4 branched alkyl, preferably one or more of tetramethyl orthosilicate, tetraethyl orthosilicate, tetra-n-propyl orthosilicate and tetra-n-butyl orthosilicate, more preferably tetraethyl orthosilicate and/or tetramethyl orthosilicate;

Optionally, the solvent is selected from deionized water and/or distilled water;

Optionally, the alkaline template is selected from one or more of ammonia, aliphatic amine, aliphatic alcohol amine and quaternary ammonium base, preferably one or more of ammonia, ethylamine, tetrapropyl ammonium hydroxide and triethanolamine;

Optionally, the tin source is selected from one or more of stannic chloride, stannic chloride pentahydrate, stannous chloride, stannous chloride dihydrate, calcium stannate, potassium stannate, sodium stannate, lithium stannate, stannous sulfate and stannous pyrophosphate, preferably stannic chloride pentahydrate;

Optionally, the titanium source has a structure shown in formula (II) below,

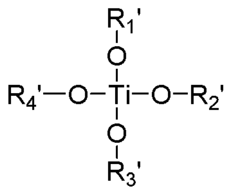

Formula (II)

In formula (II), $R_1$', $R_2$', $R_3$' and $R_4$' are each independently selected from C1-C6 alkyl, such as C1-C6 linear alkyl and C3-C6 branched alkyl; the titanium source is preferably one or more of tetramethyl titanate, tetraethyl titanate, tetraisopropyl titanate and tetrabutyl titanate.

Optionally, the hydrothermal reaction conditions in S3 include: a temperature of 120-170 °C and time of 2-5 days.

A third aspect of the invention provides an application of the tin-titanium-silicon molecular sieve provided in the first aspect of the invention in the preparation of lactate from dihydroxyacetone, an ammoximation reaction of aldehydes and ketones, an epoxidation reaction of olefins or an oxidation reaction of aromatics/alkanes.

A fourth aspect of the invention provides a catalyst containing the tin-titanium-silicon molecular sieve provided by the first aspect of the invention.

Through the above technical solutions, the tin-titanium-silicon molecular sieve of the invention has many catalytic active centers, the electron binding energy of the framework tin shifts to a lower level, and the electron cloud density increases, which help to improve the catalytic performance of the molecular sieve.

Other features and advantages of the invention will be described in detail in the following embodiment portion.

## DESCRIPTION OF THE DRAWINGS

The drawings are used to provide a further understanding of the invention and form a part of the description. They are used to explain the invention together with the following embodiments, but do not constitute a limitation of the invention. In the drawings:

Figure 1 is a XPS diagram of the tin-titanium-silicon molecular sieve prepared in Example 1 of the invention;
Figure 2 is a XPS diagram of the tin-titanium-silicon molecular sieve prepared in Comparative Example 1 of the invention.

## MODE OF CARRYING OUT THE INVENTION

The embodiments of the present invention will be described in detail below in combination with the drawings. It should be understood that the embodiments described herein are only used to illustrate and explain the invention, not to limit the invention.

A first aspect of the invention provides a tin-titanium-silicon molecular sieve. The electron binding energy of the framework tin active center in the tin-titanium-silicon molecular sieve is 488.5 eV or less. In the tin-titanium-silicon molecular sieve, the molar ratio of titanium to silicon can be 0.005-0.03, preferably 0.005-0.02, and/or the molar ratio of tin to silicon can be 0.005-0.025, preferably 0.005-0.02.

According to the invention, the framework tin refers to the tin atom that can be inserted into the molecular sieve framework. The tin-titanium-silicon molecular sieve of the invention also contains a framework titanium on the basis of comprising the framework tin, which can further increase the type and the quantity of Lewis acid of the molecular sieve and increase the catalytic active centers. Moreover, the electron binding energy of the framework tin in the molecular sieve of the invention is lower, and the density of electron cloud is increased, which is beneficial to improve the catalytic performance, especially to improve the catalytic performance of molecular sieves for the preparation of lactate from dihydroxyacetone, the ammoximation reaction of aldehydes and ketones, the epoxidation reaction of olefins, or the oxidation reaction of aromatics/alkanes.

In an embodiment of the invention, the electron binding energy of the active center of the framework tin is 488.0-488.4 eV.

In an embodiment of the invention, the electron binding energy of the active center of the framework titanium is 460.8-461.2 eV.

According to the invention, the external specific surface area of the tin-titanium-silicon molecular sieve can be 100-150 $m^2/g$, and the total pore volume can be 0.25-0.35 $cm^3/g$. Preferably, the external specific surface area is 100-140 $m^2/g$, and the total pore volume is 0.27-0.32 $cm^3/g$. The external specific surface area refers to the total area of the

molecular sieve per unit mass of material (except microporous surface area).

According to the invention, the tin-titanium-silicon molecular sieve can be an Sn-Ti-MFI molecular sieve or an Sn-Ti-MEL molecular sieve.

In an embodiment, the Sn-Ti-MEL molecular sieve has a structure in which long-strip grains overlap and stack, wherein the length of the grains may be 10-50 nm, and the width may be 10-30 nm. The long-strip shape means that the length-diameter ratio of the molecular sieve grains is greater than 1, preferably greater than 1.5, more preferably greater than 2. The overlapped and stacked structure of the grains refers to the irregularly stacked and overlapped morphology of the grains of the Sn-MEL molecular sieve observed by a high-resolution transmission electron microscope.

A second aspect of the invention provides a method for preparing the tin-titanium-silicon molecular sieve provided by the first aspect of the invention, said method comprising:

S1, mixing an organic silicon source, an organic base, a solvent, a tin source, a titanium source and an alkaline template to obtain a first mixture, wherein the organic base is tetrapropyl ammonium hydroxide and/or tetrabutyl ammonium hydroxide; the molar ratio of the amounts of the organic silicon source, the organic base, the solvent, the tin source and the titanium source is 1: (0.05-0.6): (10-30): (0.005-0.04): (0.005-0.04), wherein the organic silicon source is calculated based on $SiO_2$, the tin source is calculated based on $SnO_2$, and the titanium source is calculated based on $TiO_2$; the molar ratio of the amounts of the tin source and the alkaline template is 1: (1-10), wherein the tin source is calculated based on anion;
S2, heating the first mixture at 30-80 °C for 2-10 hours to obtain a second mixture;
S3, subjecting the second mixture to a hydrothermal reaction at 120-200 °C for 2-7 days to obtain a third mixture;
S4, taking out a solid in the third mixture and subjecting it to drying and calcination.

The method of the invention can increase the molar ratio of tin to silicon and the molar ratio of titanium to silicon in the tin-titanium-silicon molecular sieve, make the electron binding energy of the framework tin shift to a lower level, and increase the density of the electron cloud. The prepared tin-titanium-silicon molecular sieve has an excellent catalytic performance for the preparation of lactate from dihydroxyacetone, the ammoximation reaction of aldehydes and ketones, the epoxidation reaction of olefins or the oxidation reaction of aromatics/alkanes.

According to the invention, in step S1, the molar ratio of the amounts of the organic silicon source, the organic base, the solvent, the tin source and the titanium source can be 1: (0.1-0.4): (15-30): (0.01-0.03): (0.01-0.03), wherein the organic silicon source is calculated based on $SiO_2$, the tin source is calculated based on $SnO_2$, and the titanium source is calculated based on $TiO_2$.

According to the invention, the molar ratio of the amounts of the tin source and the alkaline template is preferably 1: (4-8), and more preferably, the molar ratio of the amounts of the tin source and the alkaline template is 1: (4-6), wherein the tin source is calculated based on anion.

In a preferred embodiment, step S1 can comprise: mixing the organic silicon source, the organic base, the solvent and the titanium source to obtain a fourth mixture, mixing the resulting fourth mixture with the tin source and stirring for 0.5-2 hours, and then adding the alkaline template to obtain the first mixture.

According to the invention, step S2 can comprise: heating the first mixture at 50-80 °C for 5-8 hours under stirring conditions to obtain a second mixture. Stirring can be a conventional means adopted by those skilled in the art, such as mechanical stirring, electromagnetic stirring, etc.

According to the invention, in step S4, the method of taking out the solid in the third mixture is not specifically limited. For instance, filtration, centrifugation and the like may be used. Preferably, the solid as taken out is washed before drying and calcination. There is no restriction on the solution used for washing. It can be any liquid that does not react with the solid, such as deionized water. Drying and calcination are operations familiar to those skilled in the art. In an embodiment, drying can be carried out in a constant temperature drying oven, and the drying conditions can include: a temperature of 50-150 °C, time of 2-10 hours, preferably 3-10 hours; the calcination can be conducted in a muffle furnace or a tubular furnace, and the calcination conditions can include: a temperature of 550-750 °C, time of 3-10 hours. The atmosphere is air or inert atmosphere, wherein the inert atmosphere contains nitrogen and/or inert gas that can be argon, helium, etc.

According to the invention, the organic silicon source can have a structure shown in formula (I) below,

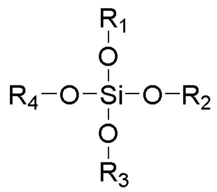

Formula (I)

In formula (I), $R_1$, $R_2$, $R_3$ and $R_4$ are each independently selected from C1-C4 alkyl that can comprise C1-C4 linear alkyl and C3-C4 branched alkyl, such as methyl, ethyl, n-propyl, isopropyl, n-butyl, sec-butyl, isobutyl or tert-butyl. In a preferred embodiment, the organic silicon source is one or more of tetramethyl orthosilicate, tetraethyl orthosilicate, tetra-n-propyl orthosilicate and tetra-n-butyl orthosilicate, more preferably tetraethyl orthosilicate and/or tetramethyl orthosilicate.

According to the invention, the titanium source can have a structure shown in formula (II) below,

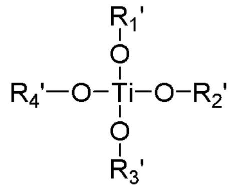

Formula (II)

In formula (II), $R_1$', $R_2$', $R_3$' and $R_4$' are each independently selected from C1-C6 alkyl that can comprise C1-C6 linear alkyl and C3-C6 branched alkyl; in a preferred embodiment, the titanium source is one or more of tetramethyl titanate, tetraethyl titanate, tetraisopropyl titanate and tetrabutyl titanate.

According to the invention, the alkaline template may be selected from one or more of ammonia, aliphatic amine, aliphatic alcohol amine and quaternary ammonium base, preferably one or more of ammonia, ethylamine, tetrapropyl ammonium hydroxide and triethanolamine. Therein the aliphatic amine can be a compound formed after at least one hydrogen in various $NH_3$ is substituted with an aliphatic hydrocarbon group (preferably alkyl), the aliphatic alcohol amine can be a compound formed after at least one hydrogen in various $NH_3$ is substituted with a hydroxyl-containing aliphatic hydrocarbon group (preferably alkyl).

In an embodiment, the quaternary ammonium base can be an organic quaternary ammonium base, which has the structure shown in formula (III)

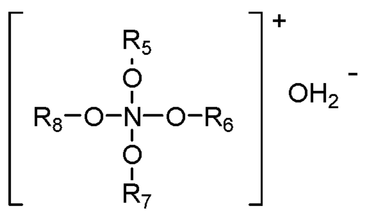

formula (III),

wherein $R_5$, $R_6$, $R_7$ and $R_8$ are each independently alkyl with a carbon atom number of 1-4, which can include a linear alkyl with a carbon atom number of 1-4 and a branched alkyl with a carbon atom number of 3-4, such as methyl, ethyl, n-propyl, isopropyl, n-butyl, sec-butyl, isobutyl or tert-butyl.

In an embodiment, the aliphatic amine has the structure shown in formula (IV), $R^9(NH_2)_n$ formula (IV), wherein n is 1 or 2. When n is 1, $R^9$ can be alkyl with a carbon atom number of 1-6, which can include a linear alkyl with a carbon atom number of 1-6 and a branched alkyl with a carbon atom number of 3-6, such as methyl, ethyl, n-propyl, isopropyl, n-butyl, sec-butyl, isobutyl, tert-butyl, n-amyl, neopentyl, isopentyl. When n is 2, $R^9$ can be alkylene with a carbon atom number of 1-6, which can include a linear alkylene with a carbon atom number of 1-6 and a branched alkylene with a carbon atom number of 3-6, such as methylene, n-propylene, n-butylene, n-pentylene. In a preferred embodiment, the aliphatic amine compound is one or more of ethylamine, n-butylamine and butanediamine.

In an embodiment, the aliphatic alcohol amine has the structure shown in formula (V), $(HOR^{10})_m(NH)_{3m}$ formula (V), wherein m is 1, 2 or 3, and m $R^{10}$ can be the same or different, each independently selected from alkylene with 1-4 carbon atoms. The alkylene can include a linear alkylene with 1-4 carbon atoms and a branched alkylene with 3-4 carbon atoms, such as methylene, ethylene, n-propylene and n-butylene. In a preferred embodiment, the aliphatic alcohol amine compound is one or more of monoethanolamine, diethanolamine and triethanolamine.

According to the invention, the solvent can be selected from deionized water and/or distilled water.

According to the invention, the tin source can be any water-soluble inorganic tin salt, preferably one or more of stannic chloride, stannic chloride pentahydrate, stannous chloride, stannous chloride dihydrate, calcium stannate, potassium stannate, sodium stannate, lithium stannate, stannous sulfate and stannous pyrophosphate, more preferably stannic chloride pentahydrate.

According to the present invention, the hydrothermal reaction is well known to those skilled in the art. For example, it can be carried out in an autoclave. There is no specific restriction on the pressure of the hydrothermal reaction, which can be either a self-generated pressure of the reaction or a pressure added additionally to the reaction system. In an embodiment, the hydrothermal reaction conditions can include: the temperature of 120-170 °C, the time of 2-5 days.

A third aspect of the invention provides an application of the tin-titanium-silicon molecular sieve provided in the first aspect of the invention in the preparation of lactate from dihydroxyacetone, the ammoximation reaction of aldehydes and ketones, the epoxidation reaction of olefins or the oxidation reaction of aromatics/alkanes.

According to the present invention, lactate is prepared using dihydroxyacetone in the presence of a solvent and the tin-titanium-silicon molecular sieve. The solvent used to prepare lactate from dihydroxyacetone is well known to those skilled in the art. For example, it can be a lower alcohol, and more specifically, it can be methanol, ethanol, propanol, butanol, etc.

In an embodiment, the invention provides an application of the tin-titanium-silicon molecular sieve in the preparation of methyl lactate from dihydroxyacetone, and the solvent is methanol.

For the ammoximation reaction of aldehydes and ketones, the solvent used is well known to those skilled in the art. For example, it can be a lower alcohol, more specifically methanol, ethanol, isopropanol, tert-butanol, etc.

For the epoxidation reaction of olefins, the solvent used is familiar to those skilled in the art. For example, it can be a lower alcohol, more specifically methanol, ethanol, isopropanol, tert-butanol, etc.

For the oxidation reaction of aromatics/alkanes, the solvent used is familiar to those skilled in the art. For example, it can be a lower alcohol, more specifically methanol, ethanol, isopropanol, tert-butanol, etc.

A fourth aspect of the invention provides a catalyst containing the tin-titanium-silicon molecular sieve provided by the first aspect of the invention.

The present invention is further illustrated by the following examples, but the present invention is not limited thereto.

Unless otherwise specified, the raw materials used in the examples and the comparative examples are all chemically pure reagents. Tetraethyl orthosilicate is purchased from Hunan Jianchang Petrochemical Co., Ltd., tetrabutyl titanate from Hunan Jianchang Petrochemical Co., Ltd., tetrabutyl ammonium hydroxide from Beijing Innochem Science & Technology Co., Ltd., stannic chloride pentahydrate from Beijing Innochem Science & Technology Co., Ltd., tetrapropyl ammonium hydroxide from Beijing Innochem Science & Technology Co., Ltd., dihydroxyacetone from Beijing Innochem Science & Technology Co., Ltd., methanol from Beijing Innochem Science & Technology Co., Ltd., stannous chloride from Beijing Innochem Science & Technology Co., Ltd., stannous chloride dihydrate from Beijing Innochem Science & Technology Co., Ltd., triethanolamine from Beijing Innochem Science & Technology Co., Ltd., and ethylamine from Beijing Innochem Science & Technology Co., Ltd.

The electron binding energy of the framework tin active center in the molecular sieve can be obtained by XPS characterization;

The electron binding energy of the framework titanium active center in the molecular sieve can be obtained by XPS characterization;

The double-quantum nuclear magnetic analysis uses trimethylphosphine as a probe molecule. After a sample is pretreated, trimethylphosphine is adsorbed on the molecular sieve, and then a measurement is performed through $^{31}P$ and $^{1}H$ nuclear magnetic resonance spectrogram. The obtained spectrogram is correlated with $^{31}P$-$^{31}P$ or $^{31}P$-$^{1}H$ to obtain said double-quantum nuclear magnetic analysis spectrogram;

The molar ratio of titanium to silicon and the molar ratio of tin to silicon in the molecular sieve are measured by XRF method;

The external specific surface area and the total pore volume of the molecular sieve are measured using BET characterization;

The length and the width of grains are characterized by a transmission electron microscope with high resolution (resolution of 50 nm).

The yield of methyl lactate in the test examples is analyzed by gas chromatography. The analysis results are

quantified by an internal standard method and the internal standard is naphthalene. The analysis conditions of chromatography are: Agilent-6890 type chromatograph, HP-5 capillary column, injection volume of 0.5 μL, and injection port temperature of 280 °C; the column temperature is maintained at 100 °C for 2 min, then increased to 200 °C at a rate of 15 °C/min and maintained for 3 min; FID detector, detector temperature of 300 °C.

The yield of methyl lactate is calculated using the following formula:

$$\text{Yield of methyl lactate } \% = \text{mole number of methyl lactate in the product / mole number of dihydroxyacetone in the raw material} \times 100\%,$$

i.e., methyl lactate yield % = methyl lactate selectivity % × dihydroxyacetone conversion %.

The conversion and the selectivity are calculated by the following formulae:

$$\text{conversion } (\%) = \text{mole number of converted organic matters / mole number of input organic matters} \times 100\%,$$

$$\text{selectivity } (\%) = \text{mole number of target product / total mole number of generated product} \times 100\%.$$

**Example 1**

S1, tetraethyl orthosilicate: tetrabutyl titanate: tetrapropylammonium hydroxide: deionized water were mixed in a molar ratio of 1:0.02:0.2:20, and stirred at room temperature; during stirring of the above mixture, stannic chloride pentahydrate was added and stirred for 1 hour to obtain a colorless transparent solution (the molar ratio of tetraethyl orthosilicate to stannic chloride pentahydrate is 1:0.02), in which tetraethyl orthosilicate was calculated based on $SiO_2$, stannic chloride pentahydrate was calculated based on $SnO_2$, and tetrabutyl titanate was calculated based on $TiO_2$; then tetrapropyl ammonium hydroxide with the same molar amount of chloride ion in stannic chloride pentahydrate was added to obtain a first mixture;

S2, the first mixture was placed on a magnetic stirrer with a reaction temperature of 70 °C and heated to remove alcohol for 6 hours to obtain a second mixture;

S3, the second mixture was placed in a stainless-steel sealed reactor and crystallized at 160 °C and the self-generated pressure for 3 days to obtain a third mixture;

S4, the third mixture was filtered to take out the solid, and the solid substance obtained was washed with water followed by drying at 110 °C for 2 hours, and then calcination in air atmosphere at 550 °C for 6 hours, thereby obtaining the tin-titanium-silicon molecular sieve of the present invention, i.e., the Sn-Ti-MFI molecular sieve, whose XPS diagram is shown in Figure 1.

The specific reaction conditions for the synthesis of the tin-titanium-silicon molecular sieve are listed in Table 1. The physicochemical characterization and the activity evaluation result of the tin-titanium-silicon molecular sieve are shown in Table 2.

**Examples 2-21**

The Sn-Ti-MFI molecular sieve was prepared according to the method of Example 1. Its ratio, synthesis condition and material ratio are shown in Table 1; the physicochemical characterization and the activity evaluation result of the tin-titanium-silicon molecular sieve are shown in Table 2, and refer to Example 1 for synthesis conditions if not involved in Table 1.

**Example 22**

S1, tetraethyl orthosilicate: tetrabutyl titanate: tetrapropylammonium hydroxide: deionized water were mixed in a molar ratio of 1:0.02:0.2:20, and stirred at room temperature; during stirring of the above mixture, stannic chloride pentahydrate was added and stirred for 1 hour to obtain a colorless transparent solution (the molar ratio of tetraethyl orthosilicate to stannous chloride pentahydrate is 1:0.01), in which tetraethyl orthosilicate was calculated based on $SiO_2$, stannous chloride pentahydrate was calculated based on $SnO_2$, and tetrabutyl titanate was calculated based on $TiO_2$; then tetrapropyl ammonium hydroxide with the same amount of chloride ion in stannic chloride pentahydrate was added to obtain a first mixture;

S2, the first mixture was placed on a magnetic stirrer with a reaction temperature of 70 °C and heated to remove alcohol for 6 hours to obtain a second mixture;

S3, the second mixture was placed in a stainless-steel sealed reactor and crystallized at 160 °C and the self-generated pressure for 3 days to obtain a third mixture;

S4, the third mixture was filtered to take out the solid, and the solid substance obtained was washed with water followed by drying at 110 °C for 2 hours, and then calcination in air atmosphere at 550 °C for 6 hours, thereby obtaining the tin-titanium-silicon molecular sieve of the present invention, i.e., the Sn-Ti-MFI molecular sieve.

The specific reaction conditions for the synthesis of the tin-titanium-silicon molecular sieve are listed in Table 1. The physicochemical characterization and the activity evaluation result of the tin-titanium-silicon molecular sieve are shown in Table 2.

**Examples 23-24**

The Sn-Ti-MFI molecular sieve was prepared according to the method of Example 22. Its ratio, synthesis condition and material ratio are shown in Table 1; the physicochemical characterization and the activity evaluation result of the tin-titanium-silicon molecular sieve are shown in Table 2, and refer to Example 22 for synthesis conditions if not involved in Table 1.

**Comparative Example 1**

The Sn-Ti-MFI molecular sieve was prepared by hydrothermal method in this comparative example.

Stannic tetrachloride pentahydrate ($SnCl_4.5H_2O$) was dissolved in water. The aqueous solution was added with tetraethyl orthosilicate (TEOS) and stirred, followed by addition of tetrabutyl titanate (TBOT), addition of tetrapropyl ammonium hydroxide (TPAOH, 20% aqueous solution) and water under stirring, stirred continuously for 30 min to obtain a clear liquid with a chemical composition of 0.015 $TiO_2$: 0.03 $SnO_2$: $SiO_2$: 0.45 TPA: 35 $H_2O$, aged at 70 °C for 6 hours, and then crystallized at 433 K for 2 days. After that, the obtained solid was filtered, washed with distilled water, dried at 393 K for 5 hours, and then calcined at 823 K for 10 hours to obtain a molecular sieve sample. Therein the amount of TEOS ewas 15.31 g, the amount of TPAOH was 33.67 g, the amount of $SnCl_4.5H_2O$ was 0.38 g, the amount of TBOT was 0.38 g, and the amount of water was 39.64 g. The XPS diagram of the prepared Sn-Ti-MFI molecular sieve is shown in Figure 2.

**Comparative Example 2**

The Sn-Ti-MFI molecular sieve was prepared by a hydrothermal method in this comparative example.

Stannic tetrachloride pentahydrate ($SnCl_4.5H_2O$) was dissolved in water. The aqueous solution was added with tetraethyl orthosilicate (TEOS) and stirred, followed by addition of tetrabutyl titanate (TBOT), addition of tetrapropyl ammonium hydroxide (TPAOH, 20% aqueous solution) and water under stirring, stirred continuously for 30 min to obtain a clear liquid with a chemical composition of 0.015 $TiO_2$: 0.03 $SnO_2$: $SiO_2$: 0.45 TPA: 35 $H_2O$, aged at 70 °C for 6 hours, and then crystallized at 433 K for 2 days. After that, the obtained solid was filtered, washed with distilled water, dried at 393 K for 5 hours, and then calcined at 733 K for 10 hours to obtain a molecular sieve sample. Therein the amount of TEOS was 15.31 g, the amount of TPAOH was 42.96 g, the amount of $SnCl_4.5H_2O$ was 0.38 g, the amount of TBOT was 0.38 g, and the amount of water was 39.64 g.

**Comparative Examples 3-10**

The Sn-Ti-MFI molecular sieve was prepared according to the method of Comparative Example 1. Its ratio, synthesis condition and material ratio are shown in Table 1; the physicochemical characterization and activity evaluation result of the tin-titanium-silicon molecular sieve are shown in Table 2, and refer to Comparative Example 1 for synthesis conditions if not involved in Table 1.

**Test Examples**

(1) Preparation of methyl lactate from catalysis of dihydroxyacetone

The samples obtained in the examples and the comparative examples were used as catalysts for the catalysis reaction of dihydroxyacetone to prepare methyl lactate. The specific reaction conditions were as follows: in the slurry bed reactor, the reactant of dihydroxyacetone and the solvent of methanol were reacted at 60 °C and the self-generated pressure at a molar ratio of 1: 200, the amount of the catalyst being 3.0% by weight of the total reaction solution, and samples were taken every hour of reaction to determine the composition of the product. The calculation results are shown in Table 2.

(2) Ammoximation reaction of aldehydes and ketones

The samples obtained in the examples and comparative examples were used as catalysts, and reacted according to the formula of

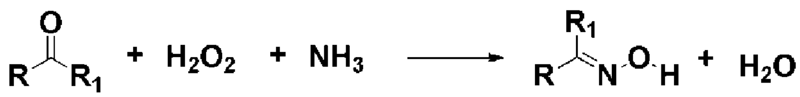

wherein R is selected from alkyl and aryl, such as phenyl; $R_1$ is selected from hydrogen and alkyl; or R and $R_1$ are combined to form a ring.

The specific reaction conditions were as follows: in the slurry bed reactor, the reactants of aldehydes and ketones, hydrogen peroxide and the solvent (such as tert-butyl alcohol) were reacted at 80 °C and the self-generated pressure at a molar ratio of 1: 1: 200, the amount of catalyst being 5.0% by weight of the total reaction solution, and samples were taken every hour of reaction to determine the composition of the product. The results are shown in Table 3.

(3) Epoxidation reaction of olefins

The samples obtained in the examples and comparative examples were used as catalysts, and reacted according to the formula of

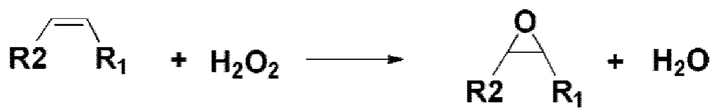

wherein $R_1$ and $R_2$ are respectively selected from hydrogen and alkyl optionally containing heteroatoms (such as oxygen), or $R_1$ and $R_2$ are combined to form a ring.

The specific reaction conditions were as follows: in the slurry bed reactor, the reactants of olefin, hydrogen peroxide and the solvent (such as tert-butyl alcohol) were reacted at 50 °C and the self-generated pressure at a molar ratio of 1: 1: 200, the amount of catalyst being 5.0% by weight of the total reaction solution, and samples were taken every hour of reaction to determine the composition of the product. The results are shown in Table 3.

(4) Oxidation reaction of aromatics/alkanes

The samples obtained in the examples and comparative examples were used as catalysts, and reacted according to the formula of

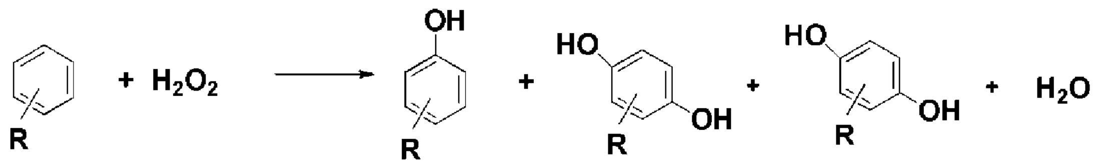

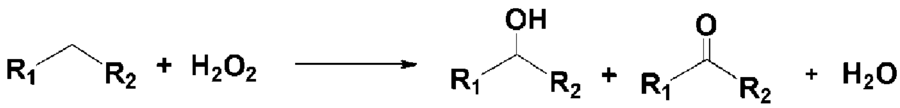

wherein R is selected from hydrogen, alkyl and hydroxyl, and R can represent two substituents; $R_1$ and $R_2$ are respectively selected from hydrogen and alkyl, or $R_1$ and $R_2$ are combined to form an optionally alkyl-substituted ring.

The specific reaction conditions were as follows: in the slurry bed reactor, the reactants (aromatics/alkanes), hydrogen peroxide and the solvent (such as tert-butyl alcohol) were reacted at 80 °C and self-generated pressure at a molar ratio of 1: 1: 200, the amount of catalyst being 5.0% by weight of the total reaction solution, and samples were taken every hour of reaction to determine the composition of the product. The results are shown in Table 3.

Table 1

| | organic silicon sources | titanium sources | inorganic tin sources | alkaline template | organic bases | organic silicon source: titanium source : organic base: solvent | organic silicon source : tin source | tin source : alkaline template | temperature and time of heating in step S2 | temperature and time of hydrothermal reaction |
|---|---|---|---|---|---|---|---|---|---|---|
| Example 1 | tetraethyl orthosilicate | tetrabutyl titanate | stannic chloride pentahydrate | tetrapropyl ammonium hvdroxide | tetrapropyl ammonium hydroxide | 1:0.02:0.2:20 | 1:0.02 | 1:1 | 70°C, 6h | 160°C,3 days |
| Example 2 | tetraethyl orthosilicate | tetrabutyl titanate | stannic chloride pentahydrate | tetrapropyl ammonium hvdroxide | tetrapropyl ammonium hydroxide | 1:0.02:0.2:20 | 1:0.02 | 1:1 | 70°C, 6h | 160°C,3 days |
| Example 3 | tetraethyl orthosilicate | tetrabutyl titanate | stannous chloride | tetrapropyl ammonium hydroxide | tetrapropyl ammonium hydroxide | 1:0.02:0.2:20 | 1:0.02 | 1:1 | 70°C, 6h | 160°C,3 days |
| Example 4 | tetraethyl orthosilicate | tetrabutyl titanate | stannous chloride dihydrate | tetrapropyl ammonium hydroxide | tetrapropyl ammonium hydroxide | 1:0.02:0.2:20 | 1:0.02 | 1:1 | 70°C, 6h | 160°C,3 days |
| Example 5 | tetraethyl orthosilicate | tetrabutyl titanate | stannic chloride pentahydrate | ammonia | tetrapropyl ammonium hydroxide | 1:0.02:0.2:20 | 1:0.02 | 1:1 | 70°C, 6h | 160°C,3 days |
| Example 6 | tetraethyl orthosilicate | tetrabutyl titanate | stannic chloride pentahydrate | ethylamine | tetrapropyl ammonium hydroxide | 1:0.02:0.2:20 | 1:0.02 | 1:1 | 70°C, 6h | 160°C,3 days |
| Example 7 | tetraethyl orthosilicate | tetrabutyl titanate | stannic chloride pentahydrate | triethanolamine | tetrapropyl ammonium hydroxide | 1:0.02:0.2:20 | 1:0.02 | 1:1 | 70°C, 6h | 160°C,3 days |
| Example 8 | tetraethyl orthosilicate | tetrabutyl titanate | stannic chloride pentahydrate | tetrapropyl ammonium hydroxide | tetrapropyl ammonium hydroxide | 1:0.02:0.1:20 | 1:0.02 | 1:1 | 70°C, 6h | 160°C,3 days |
| Example 9 | tetraethyl orthosilicate | tetrabutyl titanate | stannic chloride pentahydrate | tetrapropyl ammonium hydroxide | tetrapropyl ammonium hydroxide | 1:0.02:0.4:20 | 1:0.02 | 1:1 | 70°C, 6h | 160°C,3 days |

(continued)

| | organic silicon sources | titanium sources | inorganic tin sources | alkaline template | organic bases | organic silicon source: titanium source : organic base: solvent | organic silicon source : tin source | tin source : alkaline template | temperature and time of heating in step S2 | temperature and time of hydrothermal reaction |
|---|---|---|---|---|---|---|---|---|---|---|
| Example 10 | tetraethyl orthosilicate | tetrabutyl titanate | stannic chloride pentahydrate | tetrapropyl ammonium hydroxide | tetrapropyl ammonium hydroxide | 1:0.02:0.2:15 | 1:0.02 | 1:1 | 70°C, 6h | 160°C,3 days |
| Example 11 | tetraethyl orthosilicate | tetrabutyl titanate | stannic chloride pentahydrate | tetrapropyl ammonium hydroxide | tetrapropyl ammonium hydroxide | 1:0.02:0.2:30 | 1:0.02 | 1:1 | 70°C, 6h | 160°C,3 days |
| Example 12 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.01 | 1:1 | 70°C, 6h | 160°C,3 days |
| Example 13 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.03 | 1:1 | 70°C, 6h | 160°C,3 days |
| Example 14 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 50°C, 6h | 160°C,3 days |
| Example 15 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 80°C, 6h | 160°C,3 days |
| Example 16 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 70°C, 5h | 160°C,3 days |
| Example 17 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 70°C, 8h | 160°C,3 days |

(continued)

| | organic silicon sources | titanium sources | inorganic tin sources | alkaline template | organic bases | organic silicon source: titanium source : organic base: solvent | organic silicon source : tin source | tin source : alkaline template | temperature and time of heating in step S2 | temperature and time of hydrothermal reaction |
|---|---|---|---|---|---|---|---|---|---|---|
| Example 18 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 70°C, 6h | 120°C,3 days |
| Example 19 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 70°C, 6h | 170°C,3 days |
| Example 20 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 70°C, 6h | 160°C,2 days |
| Example 21 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 70°C, 6h | 160°C, 5 days |
| Example 22 | tetraethyl orthosilicate | tetrabutyl titanate | stannic chloride pentahydrate | tetrapropyl ammonium hydroxide | tetrabutyl ammonium hydroxide | 1:0.02:0.2:20 | 1:0.01 | 1:1 | 70°C, 6h | 160°C,3 days |
| Example 23 | tetraethyl orthosilicate | tetrabutyl titanate | stannic chloride pentahydrate | tetrapropyl ammonium hydroxide | tetrabutyl ammonium hydroxide | 1:0.02:0.2:21 | 1:0.03 | 1:1 | 70°C, 6h | 160°C,3 days |
| Example 24 | tetraethyl orthosilicate | tetrabutyl titanate | stannic chloride pentahydrate | tetrapropyl ammonium hydroxide | tetrabutyl ammonium hydroxide | 1:0.02:0.2:22 | 1:0.02 | 1:1 | 70°C, 6h | 160°C,3 days |
| Example 25 | tetraethyl orthosilicate | tetrabutyl titanate | stannic chloride pentahydrate | tetrapropyl ammonium hydroxide | tetrabutyl ammonium hydroxide | 1:0.02:0.2:20 | 1:0.02 | 1: 9 | 70°C, 6h | 160°C,3 days |

(continued)

| | organic silicon sources | titanium sources | inorganic tin sources | alkaline template | organic bases | organic silicon source: titanium source : organic base: solvent | organic silicon source : tin source | tin source : alkaline template | temperature and time of heating in step S2 | temperature and time of hydrothermal reaction |
|---|---|---|---|---|---|---|---|---|---|---|
| Example 26 | tetraethyl orthosilicate | tetrabutyl titanate | stannic chloride pentahydrate | tetrapropyl ammonium hydroxide | tetrabutyl ammonium hydroxide | 1:0.04:0.5:32 | 1:0.035 | 1:1 | 70°C, 6h | 160°C,3 days |
| Comparative Example 1 | tetraethyl orthosilicate | tetrabutyl titanate | stannic chloride pentahydrate | - | tetrapropyl ammonium hydroxide | 1:0.02:0.2:20 | 1:0.02 | 1:1 | 70°C, 6h | 160°C,3 days |
| Comparative Example 2 | tetraethyl orthosilicate | tetrabutyl titanate | stannic chloride pentahydrate | - | tetrabutyl ammonium hydroxide | 1:0.02:0.2:20 | 1:0.02 | 1:1 | 70°C, 6h | 160°C,3 days |
| Comparative Example 3 | tetraethyl orthosilicate | tetrabutyl titanate | stannic chloride pentahydrate | tetrapropyl ammonium hydroxide | tetrapropyl ammonium hydroxide | 1:0.02:0.01: 20 | 1:0.02 | 1:1 | 70°C, 6h | 160°C,3 days |
| Comparative Example 4 | tetraethyl orthosilicate | tetrabutyl titanate | stannic chloride pentahydrate | tetrapropyl ammonium hvdroxide | tetrapropyl ammonium hydroxide | 1:0.02:0.2:5 | 1:0.02 | 1:1 | 70°C, 6h | 160°C,3 days |
| Comparative Example 5 | tetraethyl orthosilicate | tetrabutyl titanate | stannic chloride pentahydrate | tetrapropyl ammonium hvdroxide | tetrapropyl ammonium hydroxide | 1:0.02:0.2:20 | 1:0.001 | 1:1 | 70°C, 6h | 160°C,3 days |
| Comparative Example 6 | tetraethyl orthosilicate | tetrabutyl titanate | stannic chloride pentahydrate | tetrapropyl ammonium hvdroxide | tetrapropyl ammonium hydroxide | 1:0.02:0.2:20 | 1:0.06 | 1:1 | 70°C, 6h | 160°C,3 days |
| Comparative Example 7 | tetraethyl orthosilicate | tetrabutyl titanate | stannic chloride pentahydrate | tetrapropyl ammonium hvdroxide | tetrapropyl ammonium hydroxide | 1:0.02:0.2:20 | 1:0.02 | 1:1 | 10°C, 6h | 160°C,3 days |

(continued)

| | organic silicon sources | titanium sources | inorganic tin sources | alkaline template | organic bases | organic silicon source: titanium source : organic base: solvent | organic silicon source : tin source | tin source : alkaline template | temperature and time of heating in step S2 | temperature and time of hydrothermal reaction |
|---|---|---|---|---|---|---|---|---|---|---|
| Comparative Example 8 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 70°C, 1h | 160°C,3 days |
| Comparative Example 9 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 70°C, 6h | 100°C, 3 days |
| Comparative Example 10 | tetraethyl orthosilicate | tetrabutyl titanate | stannic chloride pentahydrate | tetrapropyl ammonium hydroxide | tetrapropyl ammonium hydroxide | 1:0.02:0.2:20 | 1:0.02 | 1:1 | 70°C, 6h | 160°C, 1 days |

In Table 1, organic silicon source: titanium source: organic base: solvent refers to the molar ratio of the amounts of the organic silicon source, the titanium source, the organic base and the solvent; organic silicon source: tin source refers to the molar ratio of the amounts of the organic silicon source and the tin source; tin source: alkaline template refers to the molar ratio of the amounts of the tin source and the alkaline template.

Table 2

| | electron binding energy of framework tin/eV | electron binding energy of framework titanium/eV | double-quantum nuclear magnetic framework tin species/ppm | double-quantum nuclear magnetic framework titanium species /ppm | molar ratio of tin to silicon, $\times 10^{-2}$ | molar ratio of titanium to silicon, $\times 10^{-2}$ | external specific surface area, $m^2/g$ | total pore volume, $cm^3/g$ | long-strip grains | | yield of methyl lactate /% |
|---|---|---|---|---|---|---|---|---|---|---|---|
| | | | | | | | | | length, nm | width, nm | |
| Example 1 | 488.33 | 460.2 | -23.1 | -33.9 | 1.9 | 2.0 | 123 | 0.26 | 25 | 15 | 97 |
| Example 2 | 488.35 | 460.3 | -24.1 | -33.1 | 1.8 | 2.0 | 125 | 0.28 | 14 | 14 | 98 |
| Example 3 | 488.39 | 460.1 | -23.9 | -33.6 | 2.0 | 1.9 | 113 | 0.26 | 17 | 15 | 97 |
| Example 4 | 488.36 | 460.4 | -24.5 | -33.7 | 1.6 | 1.8 | 126 | 0.25 | 20 | 18 | 96 |
| Example 5 | 488.32 | 460.5 | -23.8 | -33.2 | 1.8 | 1.8 | 131 | 0.27 | 22 | 23 | 97 |
| Example 6 | 488.33 | 460.9 | -23.2 | -32.9 | 2.1 | 1.7 | 124 | 0.29 | 26 | 24 | 97 |
| Example 7 | 488.37 | 460.3 | -24.5 | -32.8 | 2.3 | 1.8 | 126 | 0.27 | 19 | 23 | 98 |
| Example 8 | 488.25 | 460.4 | -23.8 | -33.5 | 2.1 | 1.9 | 136 | 0.28 | 18 | 21 | 95 |
| Example 9 | 488.43 | 460.1 | -23.4 | -33.1 | 2.0 | 1.9 | 133 | 0.31 | 21 | 10 | 99 |
| Example 10 | 488.27 | 460.5 | -23.5 | -32.8 | 1.9 | 1.8 | 117 | 0.33 | 26 | 14 | 96 |
| Example 11 | 488.44 | 460.9 | -24.5 | -33.1 | 1.8 | 1.7 | 119 | 0.29 | 27 | 17 | 98 |
| Example 12 | 488.26 | 460.6 | -23.4 | -33.5 | 1.7 | 1.7 | 123 | 0.29 | 31 | 12 | 95 |
| Example 13 | 488.41 | 460.5 | -23.9 | -33.9 | 1.8 | 1.8 | 129 | 0.3 | 34 | 13 | 97 |
| Example 14 | 488.27 | 460.1 | -24.1 | -33.1 | 1.9 | 1.9 | 121 | 0.31 | 42 | 16 | 95 |
| Example 15 | 488.43 | 460.5 | -23.5 | -33.6 | 1.9 | 2.0 | 125 | 0.32 | 44 | 18 | 97 |
| Example 16 | 488.22 | 460.9 | -24.5 | -33.7 | 2.0 | 2.2 | 116 | 0.26 | 42 | 19 | 95 |
| Example 17 | 488.44 | 460.3 | -23.4 | -33.2 | 2.1 | 2.1 | 134 | 0.25 | 40 | 17 | 98 |
| Example 18 | 488.25 | 460.8 | -23.9 | -32.9 | 2.2 | 2.0 | 125 | 0.28 | 38 | 23 | 95 |
| Example 19 | 488.45 | 460.4 | -24.3 | -32.8 | 2.0 | 2.0 | 127 | 0.29 | 28 | 24 | 98 |
| Example 20 | 488.26 | 460.3 | -24.3 | -33.5 | 2.0 | 1.9 | 124 | 0.33 | 29 | 24 | 95 |
| Example 21 | 488.43 | 460.9 | -23.7 | -33.1 | 1.9 | 1.8 | 123 | 0.31 | 31 | 22 | 98 |
| Example 22 | 488.28 | 461.1 | -23.4 | -33.5 | 2.0 | 1.9 | 127 | 0.3 | 33 | 21 | 95 |

(continued)

| | electron binding energy of framework tin/eV | electron binding energy of framework titanium/eV | double-quantum nuclear magnetic framework tin species/ppm | double-quantum nuclear magnetic framework titanium species /ppm | molar ratio of tin to silicon, $\times 10^{-2}$ | molar ratio of titanium to silicon, $\times 10^{-2}$ | external specific surface area, $m^2/g$ | total pore volume, $cm^3/g$ | long-strip grains | | yield of methyl lactate /% |
|---|---|---|---|---|---|---|---|---|---|---|---|
| | | | | | | | | | length, nm | width, nm | |
| Example 23 | 488.46 | 460.7 | -24.2 | -33.1 | 2.0 | 2.8 | 122 | 0.3 | 36 | 20 | 97 |
| Example 24 | 488.33 | 460.5 | -23.8 | -32.8 | 1.9 | 1.9 | 131 | 0.32 | 38 | 20 | 96 |
| Example 25 | 488.50 | 460.6 | -24.3 | -33.1 | 1.8 | 1.8 | 110 | 0.22 | 26 | 29 | 93 |
| Example 26 | 488.45 | 460.8 | -23.9 | -33.5 | 1.9 | 2.9 | 123 | 0.26 | 25 | 28 | 92 |
| Comparative Example 1 | 488.69 | 459.2 | -22.8 | -34.2 | 1.9 | 1.9 | 125 | 0.24 | 12 | 2 | 75 |
| Comparative Example 2 | 488.7 | 459.3 | -22.6 | -34.7 | 1.8 | 1.8 | 127 | 0.23 | 13 | 3 | 81 |
| Comparative Example 3 | 488.59 | 459.4 | -22.4 | -34.5 | 1.8 | 1.9 | 121 | 0.25 | 18 | 6 | 78 |
| Comparative Example 4 | 488.56 | 459.1 | -21.6 | -35 | 1.9 | 1.8 | 114 | 0.22 | 14 | 8 | 77 |
| Comparative Example 5 | 488.58 | 459.6 | -21.8 | -34.6 | 2.0 | 0.09 | 115 | 0.26 | 11 | 10 | 72 |
| Comparative Example 6 | 488.52 | 459.3 | -22.1 | -34.9 | 1.9 | 2.2 | 111 | 0.27 | 12 | 6 | 79 |
| Comparative Example 7 | 488.57 | 459.2 | -21.9 | -34.8 | 1.8 | 1.8 | 107 | 0.26 | 14 | 7 | 73 |
| Comparative Example 8 | 488.58 | 459.5 | -21.4 | -34.6 | 1.9 | 1.9 | 109 | 0.25 | 13 | 8 | 74 |
| Comparative Example 9 | 488.59 | 459.1 | -22.2 | -34.6 | 1.7 | 1.8 | 104 | 0.25 | 13 | 6 | 73 |
| Comparative Example 10 | 488.58 | 459.6 | -21.8 | -34.4 | 1.8 | 1.8 | 106 | 0.25 | 14 | 9 | 75 |

Table 3

| | ammoximation reaction of aldehydes and ketones | | | epoxidation reaction of olefins | | | oxidation reaction of aromatics/alkanes | | |
|---|---|---|---|---|---|---|---|---|---|
| | raw material name | raw material conversion (%) | product selectivity (%) | raw material name | raw material conversion (%) | product selectivity (%) | raw material name | raw material conversion (%) | product selectivity (%) |
| Example 1 | acetaldehyde | 96.4 | 97.4 | propylene | 94.9 | 97.4 | phenol | 74.0 | 96.4 |
| Example 2 | benzaldehyde | 99.6 | 100.0 | ethylene | 98.0 | 98.6 | benzene | 74.9 | 97.6 |
| Example 3 | acetophenone | 98.3 | 99.3 | 1-hexene | 96.7 | 99.3 | cyclohexane | 75.5 | 98.3 |
| Example 4 | cyclohexanone | 99.1 | 100.1 | 1-octene | 97.5 | 97.8 | toluene | 74.3 | 96.8 |
| Example 5 | cyclohexanone | 98.4 | 99.4 | cyclohexene | 96.8 | 99.4 | cyclooctane | 75.5 | 98.4 |
| Example 6 | cyclopentanone | 97.5 | 98.5 | propylene | 95.9 | 98.5 | ethylbenzene | 74.8 | 97.5 |
| Example 7 | cyclooctanone | 96.8 | 97.8 | propylene | 95.3 | 97.8 | methylcyclohexene | 74.3 | 96.8 |
| Example 8 | methyl ethyl ketone | 99.2 | 98.2 | cyclooctene | 97.6 | 98.2 | phenol | 74.6 | 97.2 |
| Example 9 | acetone | 98.7 | 99.7 | methyl oleate | 97.1 | 99.7 | cresol | 75.8 | 98.7 |
| Example 10 | cyclohexanone | 99.1 | 97.6 | 1-pentene | 97.5 | 99.1 | 1-hexane | 75.3 | 98.1 |
| Example 11 | cyclohexanone | 97.9 | 98.9 | propylene | 96.3 | 98.9 | phenol | 75.1 | 97.9 |
| Example 12 | cyclooctanone | 95.7 | 96.7 | ethylene | 94.2 | 96.7 | cyclooctane | 73.5 | 95.7 |
| Example 13 | acetone | 98.4 | 99.4 | 1-hexene | 96.8 | 99.4 | ethylbenzene | 75.5 | 98.4 |
| Example 14 | acetaldehyde | 95.4 | 96.4 | 1-octene | 93.9 | 96.4 | phenol | 73.3 | 95.4 |
| Example 15 | benzaldehyde | 98.6 | 99.6 | cyclohexene | 97.0 | 99.6 | benzene | 75.7 | 98.6 |
| Example 16 | acetophenone | 97.3 | 98.3 | propylene | 95.8 | 98.3 | cyclohexane | 74.7 | 97.3 |
| Example 17 | cyclohexanone | 98.1 | 99.1 | propylene | 96.5 | 99.1 | toluene | 75.3 | 98.1 |
| Example 18 | cyclohexanone | 97.4 | 98.4 | 1-butene | 95.9 | 98.4 | cyclooctane | 74.8 | 97.4 |
| Example 19 | cyclopentanone | 96.5 | 97.5 | propylene | 95.0 | 97.5 | ethylbenzene | 74.1 | 96.5 |
| Example 20 | cyclooctanone | 95.8 | 96.8 | cyclooctene | 94.3 | 96.8 | methylcyclohexene | 73.6 | 95.8 |
| Example 21 | methyl ethyl ketone | 98.2 | 99.2 | methyl oleate | 96.6 | 99.2 | phenol | 75.4 | 98.2 |

(continued)

| | ammoximation reaction of aldehydes and ketones | | | epoxidation reaction of olefins | | | oxidation reaction of aromatics/alkanes | | |
|---|---|---|---|---|---|---|---|---|---|
| | raw material name | raw material conversion (%) | product selectivity (%) | raw material name | raw material conversion (%) | product selectivity (%) | raw material name | raw material conversion (%) | product selectivity (%) |
| Example 22 | acetone | 97.7 | 98.7 | 1-pentene | 96.1 | 98.7 | cresol | 75.0 | 97.7 |
| Example 23 | cyclohexanone | 98.1 | 99.1 | propylene | 96.5 | 99.1 | 1-hexane | 75.3 | 98.1 |
| Example 24 | cyclohexanone | 96.9 | 97.9 | 1-butene | 95.4 | 97.9 | phenol | 74.4 | 96.9 |
| Example 25 | methyl ethyl ketone | 98.9 | 99.9 | 1-pentene | 97.3 | 99.9 | cresol | 75.9 | 98.9 |
| Example 26 | acetophenone | 99.5 | 95.3 | cyclohexene | 97.9 | 97.5 | cyclohexane | 55.6 | 96.5 |
| Comparative Example 1 | acetaldehyde | 87.7 | 88.6 | propylene | 86.3 | 88.6 | phenol | 50.5 | 87.7 |
| Comparative Example 2 | benzaldehyde | 90.6 | 91.5 | ethylene | 89.2 | 91.5 | benzene | 52.2 | 90.6 |
| Comparative Example 3 | acetophenone | 89.5 | 90.3 | 1-hexene | 88.0 | 90.3 | cyclohexane | 51.5 | 87.6 |
| Comparative Example 4 | cyclohexanone | 90.2 | 91.1 | 1-octene | 88.7 | 91.1 | toluene | 51.9 | 88.4 |
| Comparative Example 5 | cyclohexanone | 89.5 | 90.4 | cyclohexene | 88.1 | 90.4 | cyclooctane | 51.6 | 87.7 |
| Comparative Example 6 | cyclopentanone | 88.7 | 89.6 | propylene | 87.3 | 89.6 | ethylbenzene | 51.1 | 86.9 |
| Comparative Example 7 | cyclooctanone | 88.1 | 89.0 | propylene | 86.7 | 89.0 | methylcyclohexene | 50.7 | 86.3 |
| Comparative Example 8 | methyl ethyl ketone | 90.3 | 91.2 | cyclooctene | 88.8 | 91.2 | phenol | 52.0 | 88.4 |
| Comparative Example 9 | acetone | 89.8 | 90.7 | methyl oleate | 88.4 | 90.7 | cresol | 51.7 | 88.0 |
| Comparative Example 10 | cyclohexanone | 90.2 | 91.1 | 1-pentene | 88.7 | 91.1 | 1-hexane | 51.9 | 88.4 |

The tin-titanium-silicon molecular sieve of the invention has more active centers, a low electron binding energy of the framework tin, and a high catalytic activity.

The preferred embodiments of the invention are described above in detail in combination with the drawings. However, the invention is not limited to the specific details in the above embodiments. Within the scope of the technical concept of the invention, a variety of simple variations of the technical solutions of the invention can be made, and these simple variations are all within the scope of protection of the invention.

In addition, it should be noted that the specific technical features described in the above embodiments can be combined in any suitable way if not contradictory. To avoid unnecessary repetition, the present invention will not further explain the various possible combinations.

Besides, various embodiments of the invention can also be combined arbitrarily. As long as they do not violate the idea of the invention, they should also be considered as the contents of the invention.

## Claims

1. A tin-titanium-silicon molecular sieve, wherein the electron binding energy of framework tin active centers in the tin-titanium-silicon molecular sieve is 488.5 eV or less.

2. The tin-titanium-silicon molecular sieve according to claim 1, wherein, in the tin-titanium-silicon molecular sieve, the molar ratio of titanium to silicon is 0.005-0.03, preferably 0.005-0.02, and/or the molar ratio of tin to silicon is 0.005-0.025, preferably 0.005-0.02.

3. The tin-titanium-silicon molecular sieve according to claim 1 or 2, wherein the electron binding energy of framework tin active centers is 488.0-488.4 eV.

4. The tin-titanium-silicon molecular sieve according to any of claims 1-3, wherein the electron binding energy of framework titanium active centers is 460.0-461.2 eV, preferably 460.8-461.2 eV.

5. The tin-titanium-silicon molecular sieve according to any of claims 1-4, wherein chemical shift data of phosphorus corresponding to double-quantum nuclear magnetic framework tin species adsorbed by a trimethylphosphine probe molecule are <_ -23 ppm; and/or chemical shift data of phosphorus corresponding to double-quantum nuclear magnetic framework titanium species adsorbed by a trimethylphosphine probe molecule are >_ -34 ppm.

6. The tin-titanium-silicon molecular sieve according to any of claims 1-5, which has an external specific surface area of 100-150 $m^2/g$, and a total pore volume of 0.25-0.35 $cm^3/g$.

7. The tin-titanium-silicon molecular sieve according to any of claims 1-6, which is an Sn-Ti-MFI molecular sieve or an Sn-Ti-MEL molecular sieve.

8. The tin-titanium-silicon molecular sieve according to claim 7, wherein the Sn-Ti-MEL molecular sieve has a structure in which long-strip grains having a length of 10-50 nm and a width of 10-30 nm overlap and stack.

9. A method for preparing the tin-titanium-silicon molecular sieve according to any of claims 1-8, said method comprising:

   S1, mixing an organic silicon source, an organic base, a solvent, a tin source, a titanium source and an alkaline template to obtain a first mixture, wherein the organic base is tetrapropyl ammonium hydroxide and/or tetrabutyl ammonium hydroxide; the molar ratio of the amounts of the organic silicon source, the organic base, the solvent, the tin source and the titanium source is 1: (0.05-0.6): (10-30): (0.005-0.04): (0.005-0.04), wherein the organic silicon source is calculated based on $SiO_2$, the tin source is calculated based on $SnO_2$, and the titanium source is calculated based on $TiO_2$; the molar ratio of the amounts of the tin source and the alkaline template is 1: (1-10), wherein the tin source is calculated based on anion;
   S2, heating the first mixture at 30-80 °C for 2-10 hours to obtain a second mixture;
   S3, subjecting the second mixture to a hydrothermal reaction at 120-200 °C for 2-7 days to obtain a third mixture;
   S4, taking out a solid in the third mixture and subjecting it to drying and calcination.

10. The method according to claim 9, wherein the molar ratio of the amounts of the tin source and the alkaline template is 1: (4-8).

**11.** The method according to claim 9 or 10, wherein the molar ratio of the amounts of the organic silicon source, the organic base, the solvent, the tin source and the titanium source is 1: (0.1-0.4): (15-30): (0.01-0.03): (0.01-0.03).

**12.** The method according to any of claims 9-11, wherein step S1 comprises: mixing the organic silicon source, the organic base, the solvent and the titanium source to obtain a fourth mixture, mixing the resulting fourth mixture with the tin source and stirring for 0.5-2 hours, and then adding the alkaline template to obtain the first mixture.

**13.** The method according to any of claims 9-12, wherein the organic silicon source has a structure shown in formula (I) below,

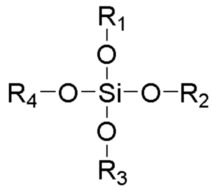

Formula (I)

wherein, $R_1$, $R_2$, $R_3$ and $R_4$ are each independently selected from C1-C4 alkyl, such as C1-C4 linear alkyl and C3-C4 branched alkyl, preferably one or more of tetramethyl orthosilicate, tetraethyl orthosilicate, tetra-n-propyl orthosilicate and tetra-n-butyl orthosilicate, more preferably tetraethyl orthosilicate and/or tetramethyl orthosilicate;
the solvent is selected from deionized water and/or distilled water;
the alkaline template is selected from one or more of ammonia, aliphatic amine, aliphatic alcohol amine and quaternary ammonium base, preferably one or more of ammonia, ethylamine, tetrapropyl ammonium hydroxide and triethanolamine;
the tin source is selected from one or more of stannic chloride, stannic chloride pentahydrate, stannous chloride, stannous chloride dihydrate, calcium stannate, potassium stannate, sodium stannate, lithium stannate, stannous sulfate and stannous pyrophosphate, preferably stannic chloride pentahydrate; and/or
the titanium source has a structure shown in formula (II) below,

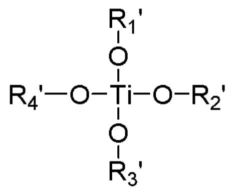

Formula (II)

wherein, $R_1$’, $R_2$’, $R_3$’ and $R_4$’ are each independently selected from C1-C6 alkyl, such as C1-C6 linear alkyl and C3-C6 branched alkyl; the titanium source is preferably one or more of tetramethyl titanate, tetraethyl titanate, tetraisopropyl titanate and tetrabutyl titanate.

**14.** The method according to any of claims 9-13, wherein the hydrothermal reaction conditions in S3 include: a temperature of 120-170 °C and time of 2-5 days.

**15.** Application of the tin-titanium-silicon molecular sieve according to any of claims 1-8 in the preparation of lactate from dihydroxyacetone, an ammoximation reaction of aldehydes and ketones, an epoxidation reaction of olefins or an oxidation reaction of aromatics/alkanes.

**16.** A catalyst comprising the tin-titanium-silicon molecular sieve according to any of claims 1-8.

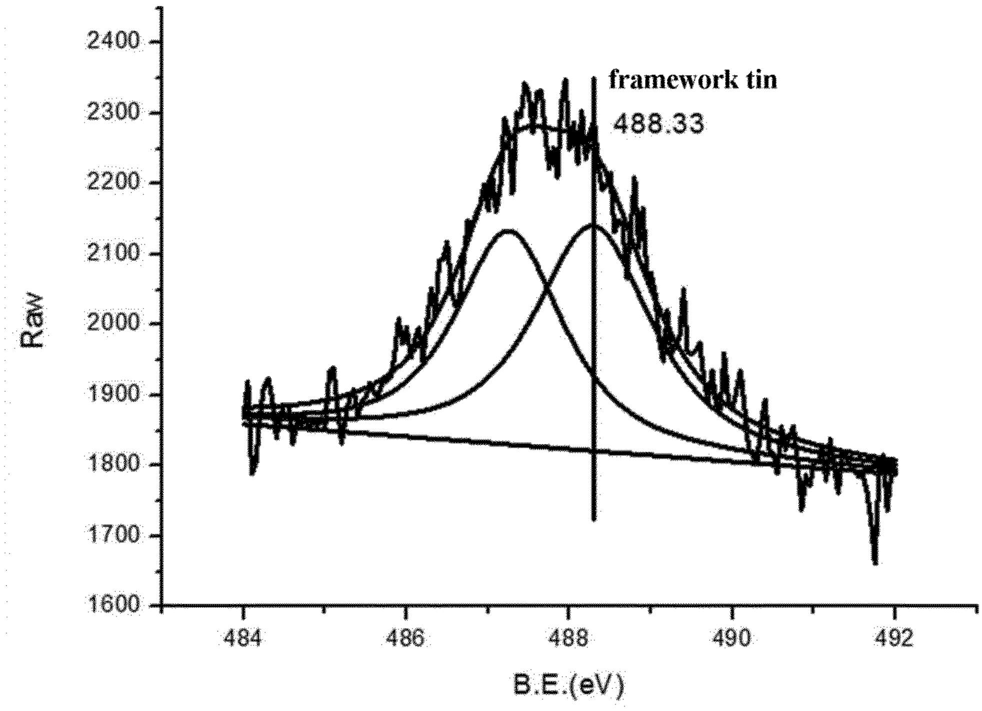
Raw
2400
2300
2200
2100
2000
1900
1800
1700
1600
484
486
488
490
492
B.E.(eV)
framework tin
488.33

Figure 1

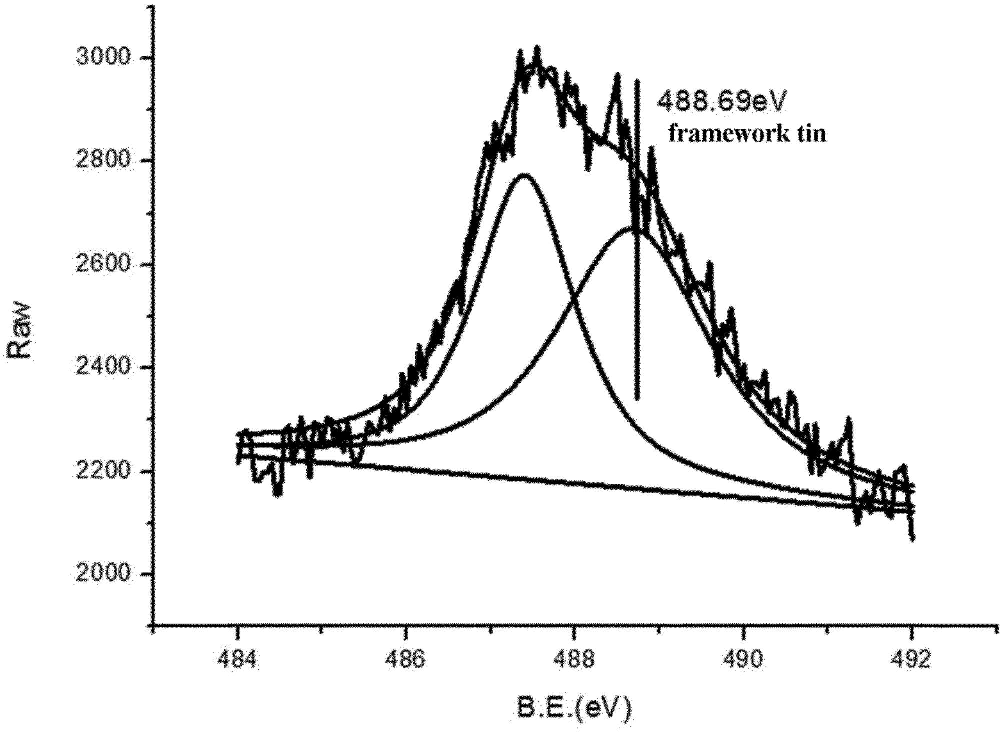

3000
2800
2600
2400
2200
2000
Raw
484
486
488
490
492
B.E.(eV)
488.69eV
framework tin


Figure 2

**INTERNATIONAL SEARCH REPORT**

International application No.
**PCT/CN2021/114731**

**A. CLASSIFICATION OF SUBJECT MATTER**

C01B 39/08(2006.01)i; C07C 69/68(2006.01)i; B01J 29/89(2006.01)i

According to International Patent Classification (IPC) or to both national classification and IPC

**B. FIELDS SEARCHED**

Minimum documentation searched (classification system followed by classification symbols)

C01B 39/-, C07C 69/-, B01J 29/-

Documentation searched other than minimum documentation to the extent that such documents are included in the fields searched

Electronic data base consulted during the international search (name of data base and, where practicable, search terms used)

CNABS, CNKI, CNTXT, VEN, WEB OF SCIENCE: 分子筛, 沸石, 锡, 钛, 硅, 比表面积, 催化剂, molecular sieve, zeolite, tin, titanium, silicon, Sn, Ti, Si, specific surface area, catalyst

**C. DOCUMENTS CONSIDERED TO BE RELEVANT**

| Category* | Citation of document, with indication, where appropriate, of the relevant passages | Relevant to claim No. |
|---|---|---|
| X | CN 111253248 A (CHINA PETROLEUM & CHEMICAL CORPORATION et al.) 09 June 2020 (2020-06-09) description, paragraphs [0006], [0008], [0015], [0019]-[0023], [0028], [0063], [0066] | 1-16 |
| X | CN 105217645 A (CHINA PETROLEUM & CHEMICAL CORPORATION et al.) 06 January 2016 (2016-01-06) claims 1-16 | 1-16 |
| X | CN 111253259 A (CHINA PETROLEUM & CHEMICAL CORPORATION et al.) 09 June 2020 (2020-06-09) claims 1-10 | 1-16 |
| X | CN 111253252 A (CHINA PETROLEUM & CHEMICAL CORPORATION et al.) 09 June 2020 (2020-06-09) claims 1-10 | 1-16 |
| X | CN 106032283 A (CHINA PETROLEUM & CHEMICAL CORPORATION et al.) 19 October 2016 (2016-10-19) claims 1-16 | 1-16 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 November 2021** | **23 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/114731**

**C. DOCUMENTS CONSIDERED TO BE RELEVANT**

| Category* | Citation of document, with indication, where appropriate, of the relevant passages | Relevant to claim No. |
|---|---|---|
| X | CN 110642263 A (CHINA PETROLEUM & CHEMICAL CORPORATION et al.) 03 January 2020 (2020-01-03) claims 1-14 | 1-16 |
| X | CN 110759353 A (CHINA PETROLEUM & CHEMICAL CORPORATION et al.) 07 February 2020 (2020-02-07) claims 1-13 | 1-16 |
| A | US 6074624 A (UOP L.L.C.) 13 June 2000 (2000-06-13) entire document | 1-16 |
| A | US 5968473 A (UOP L.L.C.) 19 October 1999 (1999-10-19) entire document | 1-16 |

Form PCT/ISA/210 (second sheet) (January 2015)

**INTERNATIONAL SEARCH REPORT**
**Information on patent family members**

International application No.
**PCT/CN2021/114731**

| Patent document cited in search report | | | Publication date (day/month/year) | Patent family member(s) | | | Publication date (day/month/year) |
|---|---|---|---|---|---|---|---|
| CN | 111253248 | A | 09 June 2020 | None | | | |
| CN | 105217645 | A | 06 January 2016 | CN | 105217645 | B | 05 January 2018 |
| CN | 111253259 | A | 09 June 2020 | None | | | |
| CN | 111253252 | A | 09 June 2020 | None | | | |
| CN | 106032283 | A | 19 October 2016 | CN | 106032283 | B | 28 September 2018 |
| CN | 110642263 | A | 03 January 2020 | CN | 110642263 | B | 12 March 2021 |
| CN | 110759353 | A | 07 February 2020 | None | | | |
| US | 6074624 | A | 13 June 2000 | None | | | |
| US | 5968473 | A | 19 October 1999 | AT | 251597 | T | 15 October 2003 |
| | | | | DE | 69911896 | T2 | 29 July 2004 |
| | | | | US | 6306364 | B1 | 23 October 2001 |
| | | | | EP | 1010667 | A1 | 21 June 2000 |
| | | | | JP | 2000178022 | A | 27 June 2000 |
| | | | | DE | 69911896 | D1 | 13 November 2003 |
| | | | | ES | 2209347 | T3 | 16 June 2004 |
| | | | | EP | 1010667 | B1 | 08 October 2003 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LI et al.** *The Journal of Physical Chemistry C,* 2011, vol. 115 (9), 3663-3670
- **HAMMOND et al.** *Angewandte Chemie International Edition,* 2012, vol. 51 (47), 11736-11739